# EUROPEAN PATENT SPECIFICATION

(11) **EP 2 142 252 B1**
(45) Date of publication and mention of the grant of the patent: **01.08.2012**
(21) Application number: 07748063.0
(22) Date of filing: 24.04.2007
(51) Int. Cl.: A61N 1/37

(54) **IMPLANTABLE HEART STIMULATING DEVICE**
IMPLANTIERBARES HERZSTIMULATIONSGERÄT
DISPOSITIF DE STIMULATION CARDIAQUE IMPLANTABLE

(43) Date of publication of application: 13.01.2010
(73) Proprietor: St. Jude Medical AB, 175 84 Järfälla (SE)
(72) Inventor: BJÖRLING, Anders, S-169 37 Solna (SE); JÄRVERUD, Karin, S-169 71 Solna (SE)
(86) International application number: PCT/SE2007/000398
(87) International publication number: WO 2008/130293

(56) References cited:
- EP-A2- 1 249 254
- US-A1- 2005 234 520
- US-B1- 6 687 545
- VAN GELDER B.M. ET AL.: 'The effect of anodal stimulation on V-V timing at varying V-V intervals' PACING AND CLINICAL ELECTROPHYSIOLOGY vol. 28, no. 8, August 2005, pages 771 - 776, XP003000369
- TAMBORERO D. ET AL.: 'Anodal capture in cardiac resynchronization therapy implications for device programming' PACING AND CLINICAL ELECTROPHYSIOLOGY vol. 29, no. 9, September 2006, pages 940 - 945, XP003019498

## Description

### Field of the invention

The present invention relates to a device according to the preambles of the independent claims, and in particular to a device for detecting anodal capture and for changing the pacing regimen of the device in order to optimize the hemodynamics of the heart.

### Background of the invention

When stimulating LV-tip to RV-ring in a biventricular system a so called anodal stimulation generating an anodal capture may occur on the RV-ring. If the left ventricle is stimulated first - which it often is - both ventricles will depolarize at the same time and a ventricle-ventricle (VV) delay optimization is then not possible to perform.

Furthermore, an automatic capture algorithm may detect loss of capture at each RV stimulation since the RV has already been stimulated and is thus refractory. This, in turn, will lead to unnecessary going into high output mode and incorrect diagnostics.

As will be discussed in detail in the following the above is related to that the unipolar voltage strength-duration curves for the LV tip and the RV ring electrodes have different shapes. Anodal thresholds are normally higher than cathodal thresholds for the same electrode. The LV thresholds are normally higher than RV thresholds and the ring thresholds are normally higher than the tip thresholds because of different surface area and distance to excitable tissue.

All these circumstances influence the bifocal stimulation thresholds so that anodal threshold may be higher than cathodal at long pulse widths, while the cathodal threshold may be higher for a short pulse width.

In order to fully explain the present invention a general background will be given in the following.

In order to excite the left ventricle, the lead must be disposed near the left ventricle, preferably in the region of the free lateral or posterior wall, which may most easily be accomplished by placing the lead through the coronary sinus and into a left cardiac vein. Unlike a lead for the right ventricle, which is disposed within the ventricle where a tip electrode can be fixed into the myocardium, the electrodes of a lead in a cardiac vein cannot be fixed into the myocardium since that would require puncturing the vein. Instead, in the case of a bipolar lead, both the tip and ring electrodes (or proximal and distal electrodes in the case where both electrodes are ring electrodes or other types of structures) are positioned within the vein adjacent to the left ventricular myocardium. Because it is fixed into the myocardium, the tip of a bipolar lead in the right ventricle has a lower capture threshold than the ring electrode. Normally, therefore, the tip of a bipolar lead is used as the cathode in order to achieve the desirable cathodal capture when a voltage pulse is impressed across the two electrodes. With a bipolar lead in a cardiac vein, on the other hand, both electrodes are external to the myocardium and may have similar capture thresholds so that either anodal or cathodal capture can occur when a pacing pulse is output through the lead. By cathodal capture is meant that cathodal stimulation is responsible for the contraction. It has been found, however, that anodal thresholds increase over time so that eventually only the desired cathodal capture will occur. Nevertheless, a problem arises when the pulse energy for a bipolar lead in a cardiac vein is adjusted. When the lead is implanted, the capture threshold for the tip or distal electrode (i.e., the electrode usually selected to function as the cathode) may be higher than that of the ring or proximal electrode. When the clinician then determines the capture threshold of the lead with a bipolar pulse in order to adjust the stimulus pulse energy, it is impossible to distinguish between anodal and cathodal capture. There is then a risk that the stimulus pulse energy will be set to an anodal capture threshold when the cathodal capture threshold is higher. As the anodal capture threshold increases over time, the stimulation pulses may no longer be of sufficient energy to excite the left ventricle (diminishing or eliminating the programmed safety margin), and the patient may experience sporadic or total loss of resynchronization therapy.

US-6,687,545 relates to a cardiac stimulation system and method for performing automatic capture verification during bipolar stimulation by eliminating capture verification during a cardiac cycle in which anodal stimulation is detected.

Anodal stimulation is detected by the absence of a delay between the bipolar stimulation pulse and an evoked response sensed at the electrode functioning as the anode during stimulation.

Automatic capture verification during bipolar stimulation is recommended only if anodal stimulation is not detected at a working stimulation output. During automatic capture verification, if anodal stimulation is detected, a capture threshold search is performed.

In US-6,687,545 unipolar sensing is performed using e.g. the right ventricular ring electrode and the housing to determine if a stimulation pulse produced anodal stimulation at the ring electrode.

According to the US-patent this is performed by determining the time from the stimulation pulse to the onset of the evoked response.

Typically, a 20 to 40 ms conduction delay to the unipolar ring evoked response signal occurs when only cathodal stimulation is present. Therefore, if there is a delay to the evoked response as determined then anodal stimulation is not indicated and will not interfere with evoked response detection during bipolar evoked response sensing of the bipolar stimulation at the currently programmed output.

If no delay to the evoked response is measured then anodal stimulation is occurring at the ring electrode at the programmed stimulation output.

Thus, the system and method disclosed in US-6,687,545 may be used to detect and to verify anodal stimulation.

US-6,611,712 relates to an apparatus and method for testing the capture threshold of a bipolar lead of a cardiac rhythm management device in order to determine an appropriate stimulus pulse energy for the lead and/or select an appropriate stimulation configuration.

The inventors have identified drawbacks related to how presently used devices react upon detected anodal capture and consequently the object of the present invention is primarily to eliminate the adverse hemodynamic consequences of anodal capture.

### Summary of the invention

The above-mentioned object is achieved by the present invention according to the independent claims.

Preferred embodiments are set forth in the dependent claims.

Generally, the present disclosure describes solutions where automatic electronic repositioning of stimulation site is performed in order to enhance hemodynamic performance, e.g. when cardiac remodelling occurs during CRT. The proposed solution is using a bipolar lead for stimulating the left ventricle and with minimum stimulation energy used (e.g. acquired through threshold search methods).

In the situations where anodal stimulation on the LV ring occurs, this is important information which may affect the device therapy and pacing settings. As an example, the pacing output can be decreased by switching polarity since the cathodal threshold is normally lower than the anodal threshold.

Furthermore, since the AV and VV delays are based on a certain activation sequence, they need to be changed for optimal pacemaker therapy if the LV stimulation site is changed from LV tip to LV ring.

The occurrence of anodal capture could be due to a change in the patient's health status-e.g. previously dormant tissue may have become viable - which may result in that anodal capture may be taken as an indication to e.g. further investigate the patient's heart failure (HF) status.

As mentioned above, the focus of the present invention is on the hemodynamic effects and health status assessment, although the saving of energy is another important benefit.

Everything described herein would also be applicable to bipolar RV leads or RA leads as well. However, the probability of anodal stimulation occurring in these leads is normally very small. Therefore, the invention is primarily described by way of example using LV leads, but is equally applicable to other types of leads.

### Short description of the appended drawings

Figure 1 shows a block diagram schematically illustrating the present invention.
Figure 2 is a block diagram illustrating a preferred embodiment of the present invention.

### Detailed description of preferred embodiments of the invention

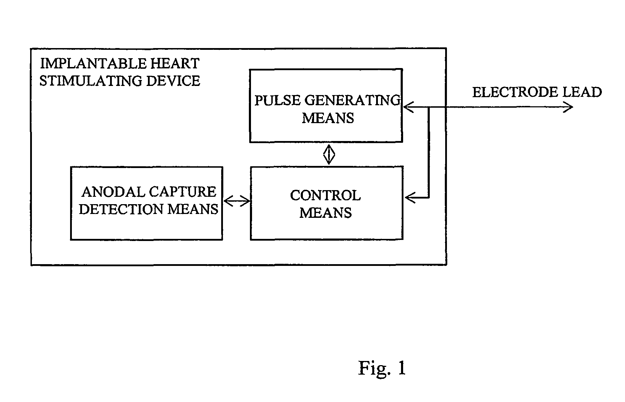

With references to figure 1 the present invention relates to an implantable heart stimulating device comprising at least one electrode lead provided with at least two electrodes (not shown) adapted to be arranged for electrical stimulation of a heart, and also for sensing electrical potentials of heart tissue. Preferably, these electrodes are adapted for stimulation/sensing of the left ventricle (LV) of the heart.

The device further comprises a pulse generating means to apply stimulation pulses between the electrodes, where one of the electrodes being the cathode and the other being the anode, to achieve cathodal capture of heart tissue by the cathode electrode, and anodal capture detection means adapted to detect anodal capture at the anode electrode.

The device further comprises a control means, wherein if anodal capture is detected by the anodal capture detection means, the control means is then arranged to change the pacing regimen in order to optimize the hemodynamics of the heart.

The pacing regime is changed by changing predefined pacing parameters being specified pacing intervals, e.g. the AV and/or the VV interval.

According to another embodiment the stimulation regime is changed by switching polarities of said electrodes and then changing said predefined pacing parameters in relation thereto.

Often, and in addition to changing the pacing regimen, a stimulation threshold search has to be performed. The person skilled in the art is familiar to numerous different types of threshold search methods that would be applicable in that respect.

Detection of anodal stimulation on e.g. the LV ring can be made either at follow-ups when the physician requests such an investigation of the capture status, or continuously during the device's operation.

According to one embodiment the anodal capture detection means is adapted to perform a so-called paced depolarization integral (PDI) initialization test in order detect anodal capture.

Studies have shown that the paced depolarization integral (PDI) is a parameter which provides important information about certain cardiac functions. This parameter is obtained, by applying a pacing pulse to the ventricle and integrating a portion of the evoked cardiac electrogram. It has been experimentally determined that the PDI parameter is proportional to a number of physiological characteristics. (See Steinhaus and Nappholz, THE INFORMATION CONTENT OF THE CARDIAC ELECTROGRAM AT THE STIMULUS SITE, Proceedings of the Twelfth Annual International Conference of the I.E.E.E. Engineering Medicine and Biology Society, Vol.12, No.2, 1990, pp. 0607-0609.)

It has been seen that during a so-called PDI initialization test which is performed in order to assess the ability to enable (biventricular) capture, anodal stimulation at the LV ring can be identified. During the PDI initialization procedure, the PDI values (areas) for different pacing amplitudes are collected and analyzed. At a pulse width of 0.5 ms, amplitudes from 0 to 4.5 V are typically investigated. In case of cathodal stimulation only, the PDI vs. amplitude plot shows two distinct plateaus - one at low amplitudes (loss of capture) and one at high amplitudes ((cathodal) capture).

In case of anodal stimulation, however, three plateaus may occur - one from loss, one from cathodal or anodal capture, and one from cathodal and anodal capture. During "double" capture, the ER morphology is different from when cathodal or anodal capture occurs and the area (PDI) is typically larger.

Not only the PDI method, but other methods as well, based on morphological features of the ER may be able to use in order to detect anodal stimulation.

In accordance with another embodiment the anodal capture detection means is adapted to measure the temporal distance between an applied stimulation pulse and a predetermined portion of the evoked response signal and anodal capture is considered detected if a predefined temporal criterion is fulfilled. This is based upon the fact that the distance from the pacing pulse to the minimum value of the ER in the unipolar signal (LV-tip to case) is longer during anodal capture than during cathodal capture. The opposite is true, i.e. the temporal distance is shorter, for the IEGM measured between the LV-ring and the case. This phenomenon may be utilized for continuous detection of anodal stimulation. See e.g. the above-referenced US-6,687,545.

As an example, if the ER morphology in the LV ring to case signal changes, particularly if the interval from stimulation to ER minimum decreases, it is likely that anodal capture has occurred. This is an example of how detection of anodal stimulation can be detected during operation of the device. Several other morphologically based detection methods may be used.

To further increase the safety of the device the anodal capture detection means comprises, according to an alternative embodiment, an anodal capture confirmation means (not shown in figure 1) adapted to confirm detected anodal capture at the anode electrode. The anodal confirmation is performed by said anodal capture confirmation means by, for one stimulation pulse, using another electrode as cathode electrode, e.g. the indifferent electrode at the housing, and by keeping the anodal electrode, and if anodal capture still occurs anodal capture is confirmed. The stimulation configuration may e.g. be changed for one stimulation pulse to LV-ring to housing electrode (LV-ring positive and housing electrode negative). If capture not is acquired for this configuration, a backup pulse may be supplied between the LV-tip and LV-ring or any other configuration to assure ventricular contraction.

However, if capture is acquired with the investigated stimulation configuration, then anodal capture is confirmed.

Irrespectively which anodal capture detection test is performed, the anodal capture detection means is arranged to automatically perform such a test continuously, at regular intervals or when specified situations occur, e.g. at follow-up procedures.

If an anodal capture is detected as a result of the test, an anodal capture alert flag is set to indicate at a follow-up procedure that anodal capture has been detected. In that case, relevant parts of a detected electrocardiogram may be stored in a storing means, included in the control means, if an anodal capture alert flag is set. The stored electrocardiogram may preferably be transferred to an external programming device to be further evaluated.

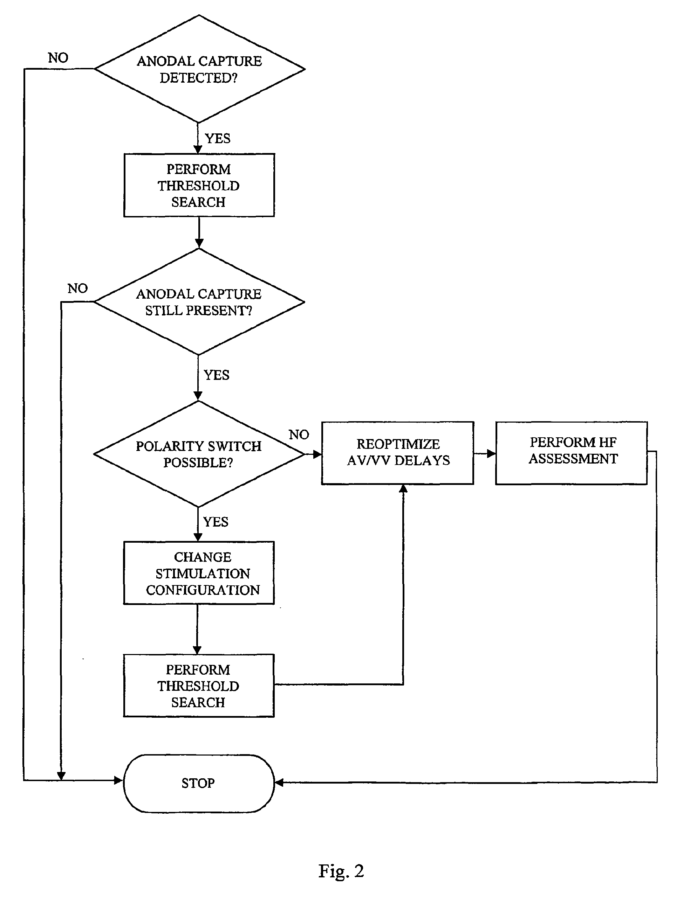

Figure 2 shows a flow diagram of one embodiment of the present invention. In particular the figure illustrates a method in an implantable heart stimulating device comprising at least one electrode lead provided with at least two electrodes adapted to be arranged for electrical stimulation of a heart. This is achieved by use of a pulse generating means adapted to apply stimulation pulses between the electrodes, where one of the electrodes being the cathode and the other being the anode, to achieve cathodal capture of heart tissue by the cathode electrode.

The implantable heart stimulating device is provided with an anodal capture detection means to detect anodal capture at the anode electrode

Upon detection of anodal capture an optional threshold search is performed and if still anodal capture is detected, the pacing regimen is changed, by the control means, in order to optimize the hemodynamics of the heart.

Preferably, it is evaluated if a polarity switch is possible. If so, the stimulation configuration is changed, e.g. the anode will become the cathode, and vice versa, and a threshold search is performed the new configuration.

In addition the pacing regimen is changed by reoptimizing specified pacing intervals, e.g. the AV and/or the VV interval. As indicated in the flow diagram of figure 2, the pacing regimen may be changed by switching polarities of the electrodes and then by changing predefined pacing parameters, e.g. specified pacing intervals, in relation thereto.

As also indicated in the flow diagram and discussed above, in addition to changing pacing regimen, irrespectively which type of change is performed, a stimulation threshold search is preferably performed using the new pacing regimen.

In order to detect anodal capture the anodal capture detection means is, according to one embodiment, adapted to perform a paced depolarization integral (PDI) initialization test (as discussed above).

According to another embodiment the anodal capture detection means is adapted to measure the temporal distance between an applied stimulation pulse and a predetermined portion of the evoked response signal and anodal capture is considered detected if a predefined temporal criterion is fulfilled, this detection method is also discussed above.

In the flow diagram is also illustrated the possibility to perform a heart failure (HF) assessment when relevant changes of the pacing regimen has been performed.

The degree of HF may automatically be assessed by the implanted device. This may be done using different methods. One of these methods is to study the patient's activity trend. By studying the output of the activity sensor of the implantable stimulator (normally used for rate responsive control) a measure of the patient's physical activity level can be acquired. This has been shown to correlate well with the degree of heart failure.

Another HF assessment tool can be to study the heart rate variability (HRV). The HRV is the variability of the patient's heart rate, which is under direct neural as well as hormonal control. The HRV has been shown to decrease with increasing levels ofHF.

A third method to assess the degree of heart failure is using impedance based upon intrathoracic impedance measurements for the early detection of pulmonary edema. This too, as well as other impedance-based methods may be used to assess the HF degree.

Other methods also exist and these three examples are intended to serve only as that, i.e. examples.

In the following one illustrative example of an implementation in accordance with the present invention is given.

Initially, a threshold search is performed on the cathode electrode. If anodal capture suddenly has appeared, this could be due to changed cellular properties. In that case, it may be reasonable to believe that the stimulation threshold at the cathode has also changed. If this is the case, the stimulation amplitude may be lowered and still achieve capture, resulting in decreased battery drain and increased longevity. Sudden anodal stimulation could thus serve as a trigger of a threshold search in devices with threshold determining capabilities.

If anodal capture disappears after this, then no further actions are performed.

However, if anodal capture still sustains after the cathodal threshold search has been performed, the polarity could be switched for additional energy savings.

In some cases it may not be possible to change polarity, e.g. due to hardware issues or the fact that the anode is in fact some kind of sensor that does not allow pacing. If that is the case, then hemodynamic settings in the device, e.g. AV and VV delay, may still be re-optimized. It is highly likely that the existing settings were optimized at the time of implantation, or at the last follow-up when only cathodal capture was present. Since both anodal and cathodal capture is now present, the depolarization sequence is somewhat changed. This, and the fact that the anodal capture could have arisen due to changed cellular properties, indicates that a re-optimization of the settings may be performed.

Furthermore, the presence of anodal capture could indicate that the patient's functional status could have changed. Thus, an additional measurement of the patient's health status (e.g. heart failure) should be obtained.

If the polarity is switched, then a new threshold search has to be performed. This time, the anode will become the cathode and vice versa. Once this is done, a re-optimization of the device settings is made and a health index measurement is obtained.

All of what is discussed above, may either be done automatically by the device, or it may be alerted to the physician at the next follow-up to perform all of the steps. It is also possible to transmit the information that anodal capture has been detected using long-range telemetry or systems like Housecall.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Implantable heart stimulating device comprising at least one electrode lead provided with at least two electrodes adapted to be arranged for electrical stimulation of a heart,
a pulse generating means adapted to apply stimulation pulses between said electrodes, one of said electrodes being the cathode and the other being the anode, to achieve cathodal capture of heart tissue by said cathode electrode,
anodal capture detection means adapted to detect anodal capture at the anode electrode, and a control means, wherein the control means is arranged to change the pacing regime of the heart, whenever said anodal capture is detected by said detection means **characterized in that** said pacing regime is changed by changing predefined pacing parameters being specified pacing intervals, e.g. the AV and/or the VV interval, in order to optimize the hemodynamics of the heart.

2. Implantable heart stimulating device according to claim 1, wherein said stimulation regime is changed by switching polarities of said electrodes and then changing said predefined pacing parameters in relation thereto.

3. Implantable heart stimulating device according to claim 1, wherein in addition to changing pacing regimen a stimulation threshold search is performed.

4. Implantable heart stimulating device according to claim 1, wherein said anodal capture detection means performs a paced depolarization integral (PDI) initialization test in order detect anodal capture.

5. Implantable heart stimulating device according to claim 1, wherein said anodal capture detection means is adapted to measure the temporal distance between an applied stimulation pulse and a predetermined portion of the evoked response signal and anodal capture is considered detected if a predefined temporal criterion is fulfilled.

6. Implantable heart stimulating device according to claim 1, wherein said anodal capture detection means comprises an anodal capture confirmation means adapted to confirm detected anodal capture at the anode electrode.

7. Implantable heart stimulating device according to claim 6, wherein said anodal confirmation is performed by said anodal capture confirmation means by, for one stimulation pulse, using another electrode as cathode electrode, e.g. the indifferent electrode at the housing, and by keeping the anodal electrode, and if anodal capture still occurs anodal capture is confirmed.

8. Implantable heart stimulating device according to claim 1, wherein said anodal capture detection means is arranged to automatically perform an anodal capture detection test continuously, at regular intervals or when specified situations occur.

9. Implantable heart stimulating device according to claim 1, wherein if an anodal capture is detected an anodal capture alert flag is set to indicate at a follow-up procedure that anodal capture has been detected.

10. Implantable heart stimulating device according to claim 9, wherein relevant parts of a detected electrocardiogram are stored in a storing means if an anodal capture alert flag is set.

11. Implantable heart stimulating device according to claim 10, wherein said stored electrocardiogram parts are transferred to an external programming device.

12. Implantable heart stimulating device according to claim 1, wherein said electrodes are adapted for stimulation of the left ventricle (LV) of the heart.

## Patentansprüche

1. Implantierbares Herzstimulationsgerät, das mindestens eine Elektrodenleitung umfasst, die mit mindestens zwei Elektroden versehen ist, die angepasst sind, zur elektrischen Stimulation eines Herzes angeordnet zu werden,
ein Impulserzeugungsmittel, das angepasst ist, Stimulationsimpulse zwischen den Elektroden abzugeben, wobei eine der Elektroden die Kathode ist und die andere die Anode ist, um eine kathodale Erregungsauslösung von Herzgewebe durch die Kathodenelektrode zu erreichen,
Mittel zum Nachweis einer anodalen Erregungsauslösung, die angepasst sind, eine anodale Erregungsauslösung an der Anodenelektrode nachzuweisen, und ein Steuermittel, wobei das Steuermittel angeordnet ist, das Stimulationsregime für das Herz immer dann zu ändern, wenn die anodale Erregungsauslösung von den Nachweismitteln nachgewiesen wird, **dadurch gekennzeichnet, dass** das Stimulationsregime durch Ändern vordefinierter Stimulationsparameter geändert wird, bei denen es sich um festgelegte Stimulationsintervalle handelt, z.B. das AV- und/oder das VV-Intervall, um die Hämodynamik des Herzes zu optimieren.

2. Implantierbares Herzstimulationsgerät nach Anspruch 1, wobei das Stimulationsregime durch Umkehren der Polarität der Elektroden und anschließendes Ändern der vordefinierten Stimulationsparameter in Bezug dazu geändert wird.

3. Implantierbares Herzstimulationsgerät nach Anspruch 1, wobei zusätzlich zur Änderung des Stimulationsregimes eine Stimulationsschwellensuche durchgeführt wird.

4. Implantierbares Herzstimulationsgerät nach Anspruch 1, wobei das Mittel zum Nachweis der anodalen Erregungsauslösung einen Initialisierungstest für das Integral der schrittmachergesteuerten Depolarisation (paced depolarization integral, PDI) durchführt, um die anodale Erregungsauslösung nachzuweisen.

5. Implantierbares Herzstimulationsgerät nach Anspruch 1, wobei das Mittel zum Nachweis der anodalen Erregungsauslösung angepasst ist, den zeitlichen Abstand zwischen einem abgegebenen Stimulationsimpuls und einem festgelegten Abschnitt des Reizantwortsignals zu messen, und die anodale Erregungsauslösung als nachgewiesen gilt, wenn ein vordefiniertes Zeitkriterium erfüllt ist.

6. Implantierbares Herzstimulationsgerät nach Anspruch 1, wobei das Mittel zum Nachweis der anodalen Erregungsauslösung ein Mittel zur Bestätigung der anodalen Erregungsauslösung umfasst, das angepasst ist, die nachgewiesene anodale Erregungsauslösung an der Anodenelektrode zu bestätigen.

7. Implantierbares Herzstimulationsgerät nach Anspruch 6, wobei die anodale Bestätigung von dem Mittel zur Bestätigung der anodalen Erregungsauslösung durch Verwenden, für einen Stimulationsimpuls, einer anderen Elektrode als Kathodenelektrode, z.B. die indifferente Elektrode am Gehäuse, und durch Beibehalten der Anodenelektrode durchgeführt wird, und wenn die anodale Erregungsauslösung weiterhin auftritt, die anodale Erregungsauslösung bestätigt ist.

8. Implantierbares Herzstimulationsgerät nach Anspruch 1, wobei das Mittel zum Nachweis der anodalen Erregungsauslösung angeordnet ist, in regelmäßigen Abständen oder wenn bestimmte Situationen eintreten, automatisch kontinuierlich einen Test zum Nachweis der anodalen Erregungsauslösung durchzuführen.

9. Implantierbares Herzstimulationsgerät nach Anspruch 1, wobei, wenn eine anodale Erregungsauslösung nachgewiesen wird, eine Warnmarkierung für die anodale Erregungsauslösung gesetzt wird, um bei einer Nachuntersuchung darauf hinzuweisen, dass eine anodale Erregungsauslösung nachgewiesen wurde.

10. Implantierbares Herzstimulationsgerät nach Anspruch 9, wobei relevante Bestandteile eines erfassten Elektrokardiogramms in einem Speichermittel gespeichert werden, wenn eine Warnmarkierung für die anodale Erregungsauslösung gesetzt ist.

11. Implantierbares Herzstimulationsgerät nach Anspruch 10, wobei die gespeicherten Elektrokardiogrammbestandteile an eine externe Programmiereinrichtung übertragen werden.

12. Implantierbares Herzstimulationsgerät nach Anspruch 1, wobei die Elektroden zur Stimulation des linken Ventrikels (LV) des Herzes angepasst sind.

## Revendications

1. Dispositif de stimulation cardiaque implantable comprenant au moins un conducteur d'électrode doté d'au moins deux électrodes adaptées pour être agencées pour la stimulation électrique d'un coeur,
un moyen de génération d'impulsions adapté pour appliquer des impulsions de stimulation entre lesdites électrodes, l'une desdites électrodes étant la cathode et l'autre étant l'anode, pour obtenir une capture cathodique de tissu cardiaque par ladite électrode cathodique,
des moyens de détection de capture anodique adaptés pour détecter une capture anodique au niveau de l'électrode anodique, et un moyen de contrôle, dans lequel le moyen de contrôle est agencé pour changer le régime de stimulation du coeur à chaque fois que ladite capture anodique est détectée par lesdits moyens de détection, **caractérisé en ce que** ledit régime de stimulation est changé en changeant des paramètres de stimulation prédéfinis qui sont des intervalles de stimulation spécifiés, par ex. les intervalles AV et/ou VV afin d'optimiser l'hémodynamique du coeur.

2. Dispositif de stimulation cardiaque implantable selon la revendication 1, dans lequel ledit régime de stimulation est changé en commutant les polarités desdites électrodes puis en changeant lesdits paramètres de stimulation prédéfinis par rapport à cela.

3. Dispositif de stimulation cardiaque implantable selon la revendication 1, dans lequel en plus de changer le régime de stimulation, une recherche de seuil de stimulation est exécutée.

4. Dispositif de stimulation cardiaque implantable selon la revendication 1, dans lequel ledit moyen de détection de capture anodique exécute un test d'initialisation d'intégrale de la dépolarisation stimulée (paced depolarization integral, PDI) afin de détecter une capture anodique.

5. Dispositif de stimulation cardiaque implantable selon la revendication 1, dans lequel ledit moyen de détection de capture anodique est adapté pour mesurer la distance temporelle entre une impulsion de stimulation appliquée et une portion prédéterminée du signal de réaction provoqué et la capture anodique est considérée comme détectée si un critère temporel prédéfini est rempli.

6. Dispositif de stimulation cardiaque implantable selon la revendication 1, dans lequel ledit moyen de détection de capture anodique comprend un moyen de confirmation de capture anodique adapté pour confirmer la capture anodique détectée au niveau de l'électrode anodique.

7. Dispositif de stimulation cardiaque implantable selon la revendication 6, dans lequel ladite confirmation anodique est exécutée par ledit moyen de confirmation de capture anodique en utilisant, pour une impulsion de stimulation, une autre électrode en tant qu'électrode cathodique, p. ex. l'électrode indifférente sur le boîtier, et en gardant l'électrode anodique, et si une capture anodique se produit toujours, la capture anodique est confirmée.

8. Dispositif de stimulation cardiaque implantable selon la revendication 1, dans lequel ledit moyen de détection de capture anodique est agencé pour exécuter automatiquement un test de détection de capture anodique en continu, à des intervalles réguliers ou quand des situations spécifiées se produisent.

9. Dispositif de stimulation cardiaque implantable selon la revendication 1, dans lequel si une capture anodique est détectée, une signalisation d'alerte de capture anodique est activée pour indiquer lors d'une procédure suivante que la capture anodique a été détectée.

10. Dispositif de stimulation cardiaque implantable selon la revendication 9, dans lequel des parties pertinentes d'un électrocardiogramme détecté sont enregistrées dans un moyen d'enregistrement si une signalisation d'alerte de capture anodique est activée.

11. Dispositif de stimulation cardiaque implantable selon la revendication 10, dans lequel lesdites parties d'électrocardiogrammes enregistrées sont transférées à un dispositif programmateur externe.

12. Dispositif de stimulation cardiaque implantable selon la revendication 1, dans lequel lesdites électrodes sont adaptées pour la stimulation du ventricule gauche (LV) du coeur.
